# FASCICULE DE BREVET EUROPEEN

(11) **EP 2 097 365 B1**
(45) Date de publication et mention de la délivrance du brevet: **16.07.2014**
(21) Numéro de dépôt: 07871942.4
(22) Date de dépôt: 14.12.2007
(51) Int. Cl.: C07C 51/25, C07C 45/52, C07C 57/055, C07C 47/22

(54) **PROCEDE DE PREPARATION D'ACIDE ACRYLIQUE A PARTIR DE GLYCEROL**
VERFAHREN ZUR HERSTELLUNG VON ACRYLSÄURE AUS GLYCEROL
METHOD FOR PREPARING ACRYLIC ACID FROM GLYCEROL

(30) Priorité: 19.12.2006 FR 0655636
(43) Date de publication de la demande: 09.09.2009
(73) Titulaire: ARKEMA FRANCE, 92700 Colombes (FR)
(72) Inventeur: DUBOIS, Jean-Luc, F-69390 Millery (FR); PATIENCE, Grégory, Ville Mont-royal, H3P 1N8 (CA)
(74) Mandataire: Bonnel, Claudine
(86) Numéro de dépôt international: PCT/FR2007/052526
(87) Numéro de publication internationale: WO 2008/087315

(56) Documents cités:
- WO-A-2006/092272
- DE-A1-102005 028 624

## Description

La présente invention concerne un procédé amélioré de préparation d'acide acrylique à partir de glycérol, comprenant une première étape de déshydratation du glycérol en acroléine et une seconde étape d'oxydation de l'acroléine en acide acrylique, dans lequel on met en oeuvre une étape intermédiaire de condensation partielle de l'eau et des sous-produits lourds issus de l'étape de déshydratation.

Le glycérol (appelé aussi glycérine) est issu de la méthanolyse des huiles végétales en même temps que les esters méthyliques qui sont, eux, employés notamment comme carburants ou combustibles dans le gazole et le fioul domestique. C'est un produit naturel, disponible en grande quantité, il peut être stocké et transporté sans difficulté. Il présente l'intérêt d'être une matière première renouvelable répondant aux critères associés au nouveau concept de "chimie verte". De nombreuses études sont consacrées à la valorisation du glycérol et la préparation de l'acide acrylique est une des voies envisagées.

La demande WO 06/114506 décrit un procédé de préparation d'acide acrylique en une étape par réaction d'oxydéshydratation du glycérol en présence d'oxygène moléculaire. Le principe du procédé est basé sur les 2 réactions consécutives de déshydratation et d'oxydation :

CH₂OH-CHOH-CH₂OH → CH₂=CH-CHO + ₂H₂O

CH₂=CH-CHO + ½ O₂ → CH₂=CH-COOH

La présence d'oxygène permet de réaliser une réaction d'oxydation, consécutivement à la réaction de déshydratation du glycérol, conduisant à la formation en une seule étape d'acide acrylique à partir du glycérol. Ce procédé peut être mis en oeuvre en phase gaz ou en phase liquide, avec des solutions aqueuses concentrées ou diluées de glycérol. Ce procédé de production d'acide acrylique directement à partir du glycérol est particulièrement avantageux puisqu'il permet la synthèse en un seul réacteur. Cependant, il est nécessaire d'introduire la totalité de l'oxygène moléculaire dès l'étage de déshydratation. Cela présente de nombreux inconvénients, notamment la réaction au cours de la première étape de déshydratation risque de s'emballer par combustion, de plus, lorsque la source d'oxygène moléculaire est l'air, le réacteur doit être beaucoup plus gros du fait de la présence de l'azote de l'air.

Dans la demande de brevet EP 1 710 227, le produit de réaction résultant de la réaction de déshydratation du glycérol en phase gaz, est soumis à une étape ultérieure d'oxydation en phase gaz pour obtenir de l'acide acrylique. Le procédé est mis en oeuvre dans deux réacteurs en série, comportant chacun un catalyseur adapté à la réaction mise en oeuvre. Il est préconisé d'ajouter de l'oxygène au mélange gazeux alimentant le second réacteur, afin d'améliorer la réaction d'oxydation et obtenir l'acide acrylique avec un rendement élevé. Ce procédé en deux étapes est mis en oeuvre avec du glycérol pur ou avec des solutions aqueuses comportant plus de 50 % en poids de glycérol. Il est conseillé d'utiliser une solution de glycérol concentrée afin de limiter le coût énergétique lié à l'évaporation de la solution aqueuse et le coût lié au traitement des eaux résiduaires. Cependant, si la concentration en glycérol est trop élevée, il risque de se produire davantage de réactions parasites, comme la formation d'éthers de glycérol, ou des réactions entre l'acroléine ou l'acide acrylique produits et le glycérol.

La demande internationale WO 2006/092272 décrit un procédé de préparation d'acide acrylique à partir de glycérol comportant soit une étape de déshydratation du glycérol en phase liquide, soit une étape de déshydratation en phase gaz. Selon l'exemple 1, le mélange réactionnel gazeux contenant l'acroléine obtenue à partir de la réaction de déshydratation du glycérol en phase gaz est mis en contact avec de l'eau dans une unité de quench avant d'être adressé au réacteur d'oxydation.

Dans le procédé de préparation d'acide acrylique à partir de glycérol décrit dans la demande internationale WO 2006/136336, le flux aqueux sortant du réacteur de déshydratation est traité de façon à recycler dans le réacteur une phase appauvrie en acroléine contenant le glycérol non réagi et alimenter le réacteur d'oxydation avec une phase enrichie en acroléine. La réaction de déshydratation est réalisée à pression élevée, notamment à une pression supérieure à 50 bars, à partir de solutions aqueuses de glycérol très diluées, en particulier contenant moins de 10% en poids de glycérol.

L'utilisation d'une solution aqueuse de glycérol dans un procédé en deux étapes présente l'inconvénient de conduire à la sortie du premier étage à un flux contenant non seulement l'acroléine produite et des sous-produits, mais aussi une quantité d'eau importante, provenant d'une part de la solution de glycérol, d'autre part de l'eau produite par la réaction de déshydratation. Ce flux alimente le second réacteur où l'acroléine est oxydée en acide acrylique en présence d'un catalyseur. Les catalyseurs classiques pour cette réaction d'oxydation sont généralement des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de nitrate, de carbonate, de sulfate ou de phosphate. Certains éléments, tels que le molybdène, le tellure ou le rhénium sont volatils, notamment en présence d'eau. Il en résulte que le catalyseur du second étage perd son efficacité et sa tenue mécanique rapidement en présence du flux de vapeur d'eau, ce qui rend difficile la maintenance du procédé. Par ailleurs, l'acide acrylique, produit en solution aqueuse diluée, nécessite des étapes de séparation et de concentration généralement compliquées et assez onéreuses.

Cependant, il a été constaté de façon surprenante que la présence d'eau dans le réacteur de déshydratation permet de favoriser la réaction de déshydratation du glycérol en phase gaz en limitant la désactivation du catalyseur de déshydratation.

Conformément à la présente invention, il est proposé un procédé amélioré de synthèse d'acide acrylique à partir de glycérol qui pallie les inconvénients des procédés précités, tout en permettant l'utilisation de solutions aqueuses diluées de glycérol produisant un effet bénéfique sur la réaction de déshydratation tout en étant économiques.

La solution qu'apporte l'invention constitue une optimisation entre la quantité d'eau alimentant le réacteur de déshydratation du premier étage et la quantité d'eau introduite dans le réacteur d'oxydation du deuxième étage. La solution consiste à condenser au moins en partie l'eau présente dans le flux issu de la réaction de déshydratation de la solution aqueuse de glycérol pour éviter, d'une part que le catalyseur du second étage ne se désactive trop rapidement, d'autre part que la solution d'acide acrylique produite soit trop diluée.

Plus précisément, la présente invention a pour objet un procédé de préparation d'acide acrylique à partir d'une solution aqueuse de glycérol, comprenant une première étape de déshydratation du glycérol en acroléine effectuée en phase gaz en présence d'un catalyseur et sous une pression comprise entre 1 et 5 bars, et une seconde étape d'oxydation de l'acroléine en acide acrylique, dans lequel on met en oeuvre une étape intermédiaire consistant à condenser au moins en partie l'eau et les sous-produits lourds présents dans le flux issu de la première étape de déshydratation.

Dans le procédé selon l'invention, condenser au moins en partie signifie que 20% à 95%, de préférence 40% à 90% de l'eau présente dans le flux issu de la première étape est éliminée dans l'étape intermédiaire avant d'alimenter le réacteur de second étage.

La réaction de déshydratation du glycérol en acroléine s'accompagne généralement de réactions secondaires entraînant la formation de sous-produits, tels que l'hydroxypropanone, le propanaldéhyde, l'acétaldéhyde, l'acétone, le phénol, des produits d'addition de l'acroléine sur le glycérol, des produits de polycondensation du glycérol, des éthers de glycérol cycliques. La condensation intermédiaire du procédé de l'invention présente en outre l'avantage de séparer, au moins en partie, les sous-produits lourds issus de ces réactions secondaires. Il en résulte une amélioration de la sélectivité de la réaction d'oxydation qui s'effectue en l'absence de ces sous-produits.

D'autres caractéristiques et avantages de l'invention ressortiront mieux à la lecture de la description qui suit et en référence à la figure annexée qui représente schématiquement un mode de réalisation de l'invention.

Dans le procédé de l'invention, on utilise une solution aqueuse de glycérol de concentration allant de 20 % à 99 % en poids dans le réacteur, de préférence entre 30 % et 80 %.

La solution de glycérol peut être utilisée sous forme liquide ou sous forme gazeuse, de préférence sous forme phase gaz.

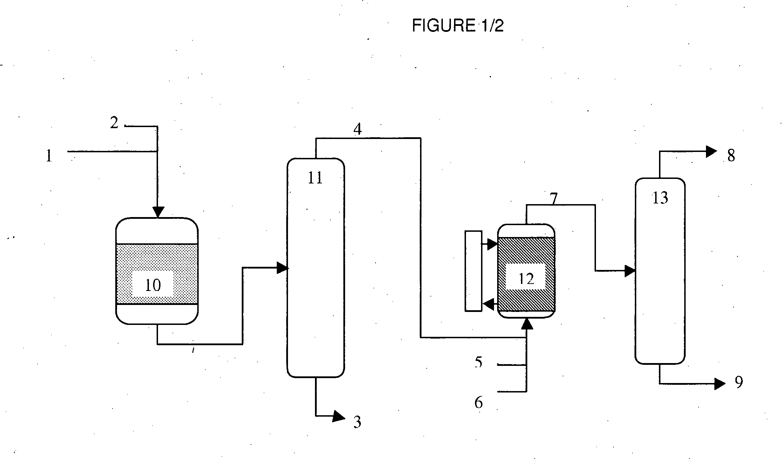

En référence à la figure 1, le glycérol est introduit en (1), dans un premier réacteur (10) de déshydratation. De l'oxygène moléculaire (2) peut être également introduit, par exemple sous forme d'air ou sous forme d'air enrichi ou appauvri en oxygène moléculaire. De préférence, la quantité d'oxygène est choisie de façon à être en dehors du domaine d'inflammabilité en tout point de l'installation. La présence d'oxygène permet de limiter la désactivation du catalyseur de déshydratation par cokage. De plus, l'ajout d'oxygène améliore le rendement de la réaction pour de nombreux systèmes catalytiques.

La réaction de déshydratation est effectuée en phase gaz dans le réacteur (10) en présence d'un catalyseur à une température allant de 150°C à 500°C, de préférence comprise entre 250°C et 350°C, et une pression comprise entre 1 et 5 bars, de préférence entre 1 et 3 bars.

Le réacteur (10) peut fonctionner en lit fixe, en lit fluidisé ou en lit fluidisé circulant, ou dans une configuration en modules (plaques ou paniers), généralement en présence de catalyseurs solides acides.

Les catalyseurs qui conviennent sont des matériaux homogènes ou multiphases, insolubles dans le milieu réactionnel qui ont une acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse. Les catalyseurs répondant au critère d'acidité H₀ inférieur à +2, peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides.

Avantageusement, les catalyseurs sont choisis parmi les zéolithes, les composites Nafion^{®} (à base d'acide sulfonique de polymères fluorés), les alumines chlorées, les acides et sels d'acides phosphotungstiques et/ou silicotungstiques, les phosphates de fer dopés par des métaux ou des métaux alcalins ou alcalino terreux FePₓM'_{y}M"_{y}O_{z}, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃. Selon les données de la littérature, ces catalyseurs ont tous une acidité de Hammett H₀ inférieure à +2.

Les catalyseurs préférés sont les zircones sulfatées, les zircones phosphatées, les zircones tungstées, les zircones silicées, les oxydes de titane ou d'étain sulfatés, les alumines ou silices phosphatées.

Ces catalyseurs ont tous une acidité de Hammett H₀ inférieure à +2, l'acidité Ho peut alors varier dans une large mesure, jusqu'à des valeurs pouvant atteindre -20 dans l'échelle de référence avec les indicateurs de Hammett. Le tableau donné à la page 71 de la publication sur la catalyse acido-basique (C. Marcilly) Vol 1 aux Editions Technip (n°ISBN 2-7108-0841-2) illustre des exemples de catalyseurs solides dans cette gamme d'acidité.

Le flux gazeux, à la sortie du réacteur (10), est constitué d'un mélange comprenant de l'acroléine, de l'eau, du glycérol non transformé et des sous-produits, tels que l'hydroxypropanone, le propanaldéhyde, l'acétaldéhyde, l'acétone, du phénol, des produits d'addition de l'acroléine sur le glycérol, des produits de polycondensation du glycérol, des éthers de glycérol cycliques ou non.

Conformément au procédé selon l'invention, ce flux est envoyé à une unité de condensation (11), qui sépare, d'une part un mélange (3) riche en eau contenant les sous-produits lourds tels que phénol, hydroxypropanone, et des produits d'addition de l'acroléine sur le glycérol (acétals), des produits de polycondensation du glycérol, des éthers de glycérol cycliques ou non, de l'acide propionique, de l'acide acrylique, de l'acide acétique, d'autre part un flux (4) riche en acroléine contenant les sous-produits légers, tels que acétaldéhyde, propanaldéhyde, acétone et éventuellement des gaz inertes, CO et CO₂.

L'unité de condensation partielle (11) peut être une colonne d'absorption couplée ou non à un évaporateur, un échangeur de chaleur, un condenseur, un déflegmateur, ainsi que tout appareillage bien connu de l'homme de l'art, permettant de réaliser une condensation partielle d'un flux aqueux. L'unité (11) peut par ailleurs être utilisée pour réchauffer la solution aqueuse de glycérol (1) alimentant le réacteur (10), optimisant ainsi le coût énergétique de l'installation.

Le flux (3) est envoyé en tout ou partie, soit vers une colonne de rectification ou de strippage dans le but de récupérer la fraction légère qui pourrait être absorbée dans ce flux, soit vers une station de traitement des eaux usées. Il peut aussi être envoyé vers un oxydeur thermique, ou encore une partie de ce flux est recyclée pour diluer le glycérol à la concentration souhaitée.

Le flux (4), riche en acroléine et débarrassé des sous-produits lourds et de l'essentiel de l'eau est envoyé dans le réacteur d'oxydation (12), où il est alors possible d'effectuer l'oxydation de l'acroléine en acide acrylique avec une pression partielle en acroléine contrôlée et plus élevée. La productivité du réacteur est ainsi améliorée.

La réaction est effectuée en présence d'oxygène moléculaire (6) qui peut être sous forme d'air ou sous forme d'air enrichi ou dilué en oxygène moléculaire, à une teneur allant de 3 à 20 % volume, par rapport au flux entrant, et éventuellement en présence de gaz inertes (5), tels que N₂, CO₂, méthane, éthane, propane ou autres alcanes légers. Les gaz inertes nécessaires au procédé peuvent éventuellement être en tout ou partie constitués de gaz (8) obtenus en tête de la colonne d'absorption (13).

La réaction d'oxydation s'effectue à une température allant de 200°C à 350°C, de préférence de 250°C à 320°C, et sous une pression allant de 1 à 5 atmosphères.

Le réacteur (12), peut fonctionner en lit fixe, en lit fluidisé ou en lit fluidisé circulant. Il est possible aussi d'utiliser un échangeur à plaques avec un agencement modulaire du catalyseur tel que décrit dans les documents EP 995491, EP 1147807 ou US 2005/0020851.

Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

L'effluent (7) issu de l'étape d'oxydation, riche en acide acrylique est ensuite purifié sur une unité de séparation (13) pour séparer d'une part les produits légers (8) de la réaction tels que le propanaldéhyde, l'acétaldéhyde, l'acétone, le CO et le CO₂, les gaz inertes de dilution et l'acroléine non convertie, et d'autre part l'acide acrylique (9) pouvant contenir encore des traces de sous-produits lourds.

Le procédé selon l'invention, même s'il nécessite un équipement supplémentaire lié à l'étape intermédiaire, présente l'avantage de mettre en oeuvre une matière première économique et de pouvoir optimiser séparément les deux étages de réaction. Il en résulte une augmentation de la productivité et de la sélectivité en acide acrylique. Le procédé reste tout à fait économique.

Comparativement à un procédé conventionnel de préparation d'acide acrylique par oxydation catalytique du propylène, le procédé selon l'invention contribue à augmenter la productivité en acide acrylique, tout en réduisant la dépendance à une ressource fossile telle que le propylène. Un tel procédé répond aux critères associés au nouveau concept de "chimie verte" dans un cadre plus global de développement durable.

### EXEMPLES

Une simulation à l'aide du logiciel ASPEN a été utilisée pour illustrer le procédé selon l'invention. Les pourcentages sont exprimés en % massiques. On ne mentionnera pas les espèces dont la teneur est inférieure à 1 %

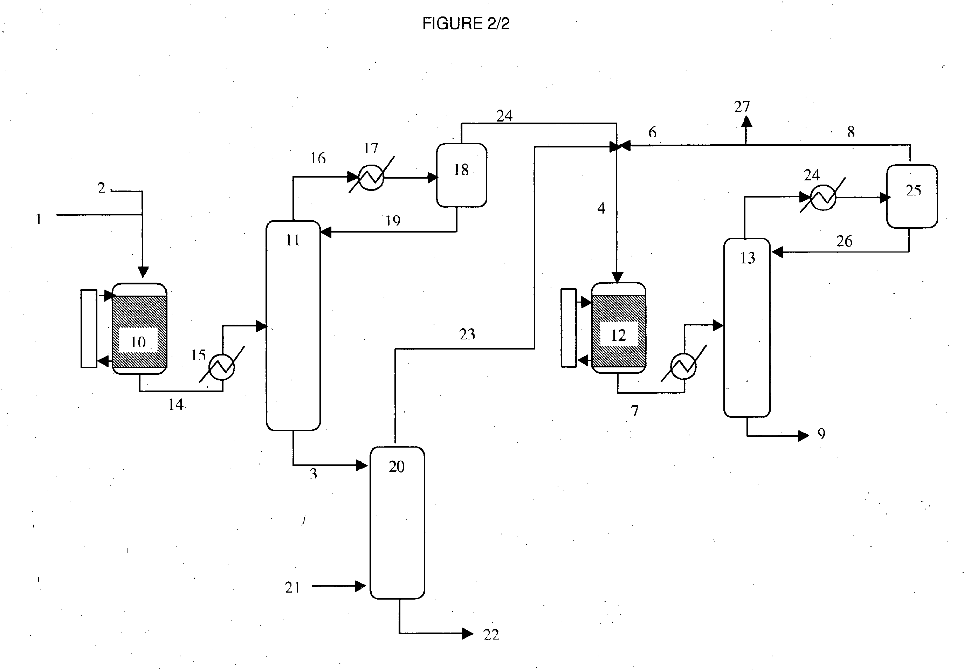

### Exemple 1 (en référence à la figure 2)

Un flux gazeux à 331°C sous 2,0 bars (50,3 T/h, 34,5% glycérol, 34,5% eau, 23,7% azote, 7,2% oxygène) est envoyé dans un réacteur multitubulaire à lit fixe (10) contenant un catalyseur hétérogène de déshydratation couplé à un bain de sel fondu. De ce réacteur sort un flux gazeux (14) à 320°C sous 1,7 bar (50,3 T/h, 47,9% eau, 23,7% azote, 5,0% oxygène, 16,4% acroléine, 1,6% acétaldéhyde, 1,4% CO, 1,1% CO₂). Ce flux est refroidi à 151°C dans un échangeur de chaleur (15) et envoyé en pied d'une colonne d'absorption (11) qui comporte 4 étages théoriques. Le flux gazeux (16) qui sort à 102°C en tête de cette colonne d'absorption est envoyé vers un condenseur partiel (17) qui le refroidit à 79°C, puis vers un pot de flash (18) qui sépare la phase gaz (24) de la phase liquide (19). Cette phase liquide (19) est renvoyée en tête de la colonne d'absorption (11). En pied de la colonne d'absorption, on soutire une phase liquide (3) à 103°C (20,4 T/h, 94,3% eau, 1,4% acide acétique, 1,0% acide formique). Cette phase liquide (3) est envoyée en tête d'une colonne de stripping (20) comportant 8 plateaux, dans laquelle on injecte en pied 4,4 T/h d'air (21) à 90°C sous 1,7 bars. En pied de cette colonne de stripping, on récupère un flux aqueux (22) (55°C, 18,6 T/h, 94,5% d'eau, 1,3% acide acétique, 1,0% acide formique). Le flux gazeux (23) récupéré en tête de la colonne de stripping est mélangé à la phase gaz (24) du pot de flash précédemment décrit (79°C, 29,9 T/h, 39,9% N₂, 27,2% acroléine, 8,4% oxygène, 16,2% eau, 2,7% acétaldéhyde, 2,4%. monoxyde de carbone, 1,8% dioxyde de carbone) et à un flux gazeux (6) (33,5 T/h, 77,2% azote, 3,6% oxygène, 7,2% eau, 5,4% dioxyde de carbone, 4,5% monoxyde de carbone). Le mélange est réchauffé à 160°C, puis injecté dans un deuxième réacteur multitubulaire (12) comportant un catalyseur d'oxydation. En sortie de ce réacteur, on obtient un flux gazeux (7) à 245°C sous 1,4 bars (69,6 T/h, 59,1% azote, 13,1% eau, 14,7% acide acrylique, 2,8% oxygène, 4,2% dioxyde de carbone, 3,4% monoxyde de carbone, 1,6% acide acétique). Ce flux est refroidi à 157°C puis injecté en pied de la colonne d'absorption (13). En tête de cette colonne, le flux gazeux est condensé partiellement dans l'échangeur de chaleur (24), puis envoyé vers un pot séparateur (25) qui produit une phase liquide (26) et une phase gaz (8) (55,6 T/h, 53°C, 77,2% azote, 3,6% oxygène, 7,2% eau, 5,4% dioxyde de carbone, 4,5% monoxyde de carbone). La phase liquide est renvoyée vers la colonne (13). La phase gaz est partiellement recyclée en amont du réacteur (12) via le flux (6). En pied de la colonne d'absorption (13), on obtient un flux (9) concentré en acide acrylique (15,6 T/h, 64,1% acide acrylique, 34,4% eau).

On notera que le procédé permet d'éliminer dans la phase aqueuse (22) certaines impuretés produites dans le réacteur de déshydratation (10) : par exemple les débits d'hydroxypropanone et d'acide acétique dans le flux gazeux en sortie du réacteur de déshydratation (10) sont respectivement de 83,7 et 254 kg/h. Ils sont de 82,2 et 237 kg/h dans le flux aqueux (22) en pied de la colonne de stripping et de 1,5 et 18 kg/h à l'entrée du réacteur d'oxydation (12).

## Revendications

1. Procédé de préparation d'acide acrylique à partir d'une solution aqueuse de glycérol, comprenant une première étape de déshydratation du glycérol en acroléine effectuée en phase gaz en présence d'un catalyseur et sous une pression comprise entre 1 et 5 bars, et une seconde étape d'oxydation de l'acroléine en acide acrylique, dans lequel on met en oeuvre une étape intermédiaire consistant à condenser au moins en partie l'eau et les sous-produits lourds présents dans le flux issu de la première étape de déshydratation.

2. Procédé selon la revendication 1, caractérisé en que l'on utilise une solution aqueuse de glycérol de concentration allant de 20 % à 99 % en poids dans le réacteur, de préférence entre 30 % et 80 %.

3. Procédé selon la revendication 1 ou 2, **caractérisé en ce que** l'on ajoute de l'oxygène moléculaire pour l'étape de déshydratation du glycérol.

## Patentansprüche

1. Verfahren zur Herstellung von Acrylsäure aus einer wässrigen Lösung von Glycerin, umfassend einen ersten Schritt der Dehydratisierung von Glycerin zu Acrolein, der in der Gasphase in Gegenwart eines Katalysators und unter einem Druck zwischen 1 und 5 bar durchgeführt wird, und einen zweiten Schritt der Oxidation des Acroleins zu Acrylsäure, bei dem ein Zwischenschnitt durchgerührt wird, der aus der zumindest teilweisen Kondensation des Wassers und der in dem Strom aus dem ersten Dehydratisierungsschritt vorliegenden schweren Nebenprodukte besteht.

2. Verfahren nach Anspruch 1, **dadurch gekennzeichnet, dass** man eine wässrige Lösung von Glycerin mit einer Konzentration im Bereich von 20 bis 99 Gew.-% und vorzugsweise zwischen 30 und 80 Gew.-% im Reaktor verwendet.

3. Verfahren nach Anspruch 1 oder 2, **dadurch gekennzeichnet, dass** man für den Schritt der Dehydratisierung von Glycerid molekularen Sauerstoff zugibt.

## Claims

1. Method for preparing acrylic acid from an aqueous glycerol solution, comprising a first step of dehydration of the glycerol to acrolein, carried out in the gas phase in the presence of a catalyst and under a pressure of between 1 and 5 bar, and a second step of oxidation of the acrolein to acrylic acid, in which an intermediate step is implemented, consisting in at least partly condensing the water and heavy by-products present in the stream issuing from the first dehydration step.

2. Method according two Claim 1, **characterized in that** use is made of an aqueous glycerol solution having a concentration of between 20% and 99% by weight in the reactor, preferably between 30% and 80%.

3. Method according to either of Claims 1 and 2, **characterized in that** molecular oxygen is added in the glycerol dehydration step.
